# EUROPEAN PATENT APPLICATION

(11) **EP 4 163 372 A1**
(43) Date of publication of application: **12.04.2023**
(21) Application number: 20938589.7
(22) Date of filing: 26.06.2020
(51) Int. Cl.: C12N 15/113, A61K 47/30, A61K 47/44, A61K 47/42

(54) **NUCLEIC ACID MOLECULE HAVING IMPROVED STABILITY, AND USE THEREOF**

(30) Priority: 04.06.2020 KR 20200067831
(71) Applicant: ImNewRun, Inc., Suwon-si, Gyeonggi-do 16419 (KR)
(72) Inventor: KIM, Yong Ho, Suwon-si, Gyeonggi-do 16419 (KR); LEE, Jae Cheol, Suwon-si, Gyeonggi-do 16419 (KR); SUH, Minah, Suwon-si, Gyeonggi-do 16419 (KR); NAM, Ji Young, Suwon-si, Gyeonggi-do 16419 (KR); LEE, Soo Yeon, Suwon-si, Gyeonggi-do 16419 (KR); LEE, Euna, Suwon-si, Gyeonggi-do 16419 (KR); LEE, Bok Soo, Suwon-si, Gyeonggi-do 16419 (KR); KIM, Han Joo, Suwon-si, Gyeonggi-do 16419 (KR)
(74) Representative: Lavoix
(86) International application number: PCT/KR2020/008398
(87) International publication number: WO 2021/246565

(57) **Abstract**

Provided are structural properties of double-stranded RNA molecules for inducing RNA interference, and more specifically, a novel nucleic acid molecule structure for inducing RNAi with improved stability, and a use thereof. A nucleic acid molecule for inducing RNAi provided herein is capable of significantly improving stability of a double-stranded RNA molecule *in vivo* while maintaining inhibition efficiency for a target gene, and therefore, as a expandable platform for targeting various genes, the RNA nucleic acid molecule is expected to replace siRNA molecules in the art, and be usefully utilized in the field of diagnosis or treatment of various diseases in research and clinical fields.

## Description

### TECHNICAL FIELD

The present disclosure relates to structural properties of double-stranded RNA molecules for inducing RNA interference, and more specifically, to a novel nucleic acid molecule structure for inducing RNAi with improved stability and a use thereof.

### BACKGROUND ART

RNA interference (RNAi) is an endogenous mechanism that regulates gene expression after transcription in a sequence-specific manner by a double-stranded RNA (dsRNA) composed of a sense strand having a sequence homologous to a target gene, and an antisense strand having a sequence complementary thereto. This mechanism has been commonly conserved in eukaryotes from *C. elegans* to plants, fruit flies, and mammals. RNA interference is known to occur as follows. When dsRNA enters a cell, dsRNA binds to an RNA-induced silencing complex (RISC), and then, only the antisense (guide) strand binds to mRNA sequence-complementarily, and the target mRNA is degraded by an endonuclease domain present in the RISC complex.

Small interfering RNA (siRNA) is a double-stranded RNA of about 19 to 29 nucleotides in length, and is capable of specifically degrading mRNA of a target gene through the RNAi pathway. Also, siRNA has such high specificity and easy access to various target genes, and in addition, one siRNA molecule can degrade as many as 1,000 mRNAs, and thus, siRNA has been considered as a very effective means to inhibit expression of target genes. Due to these advantages, siRNA has been evaluated for its effectiveness as a gene therapy agent through various clinical trials, and in August 2018, Alnylam's siRNA drug (Patisiran) was approved by the US FDA as the first RNA interference drug for the treatment of polyneuropathy in adult patients with hereditary transthyretin-mediated (hATTR) amyloidosis. However, despite the high therapeutic potential of siRNA, there are still challenges to be met, such as low stability for being easily degraded by enzymes in the blood and rapidly eliminated by the kidney due to its small size, and lack of an efficient targeted delivery system.

In order to solve these issues, various non-viral carriers such as liposomes, lipids, and polymers have been extensively studied, and in particular, it has been found that cationic polymers are easily bound to siRNA due to being soluble in water and having a high positive charge and thus reduce degradation of siRNA from various RNases distributed in the blood, thus increasing delivery efficiency of siRNA. However, cationic substances are limited in clinical application due to their potential cytotoxicity and induction of immune responses. Therefore, alternative strategies have been developed to deliver siRNA to target cells by directly conjugating various moieties such as lipids, peptides, polymers, or proteins to siRNA.

On the other hand, peptide nucleic acid (PNA) is an oligonucleotide analog, in which the deoxyribose backbone of DNA is substituted with a pseudo-peptide backbone (Nieslsen et al., Science, 1991, 254, 1475), each subunit or monomer present in PNA has a natural or unnatural nucleobase attached to the backbone. PNA has advantages of exhibiting high affinity for complementary DNA and RNA, resistance to nucleases and proteases, and high stability in a biological environment due to these structural changes. In addition, the PNA/DNA or PNA/RNA duplex exhibits higher thermodynamic stability as compared to the corresponding DNA/DNA or RNA/RNA duplex, as determined by the melting temperature (Tm).

### DESCRIPTION OF EMBODIMENTS

### TECHNICAL PROBLEM

As a result of research efforts of the present inventors to develop a method that may significantly improve *in vivo* stability without significantly affecting target gene silencing efficiency, a novel RNAi-inducing nucleic acid molecule structure in which PNA and a nucleic acid molecule are combined was developed, and based on this, the present disclosure was completed.

Accordingly, an object of the present disclosure is to provide a nucleic acid molecule for inducing RNAi, including double-stranded RNA molecule; and PNA bound to the RNA molecule.

In addition, another object of the present disclosure is to provide a complex for inducing RNAi, including the nucleic acid molecule, and an intracellular carrier.

In addition, still another object of the present disclosure is to provide a composition for gene silencing, including the complex.

However, technical problems to be solved by the present disclosure is not limited to the above-mentioned problems, and other problems not mentioned will be clearly understood by those skilled in the art from the following description.

### SOLUTION TO PROBLEM

In order to achieve the objects of the present disclosure as described above, the present disclosure provides a nucleic acid molecule for inducing RNAi, including: a double-stranded RNA molecule including a first strand, which is 15 to 49 nucleotides in length and including a region complementary to a target nucleic acid, and a second strand, which is 21 to 62 nucleotides in length and forming a complementary bond with the first strand; and a peptide nucleic acid (PNA), which is 6 to 13 nucleotides in length and bound to the first strand, wherein
the N-terminus of the PNA is joined to the 3' end of the first strand, and
the binding region between the PNA and the first strand consists of guanine (G) or cytosine (C) bases.

In an embodiment of the present disclosure, the binding region may include at least one region selected from the group consisting of a region of 1 to 2 nucleotides from the 3' end of the first strand, and a region of 1 to 2 nucleotides from the N-terminus of the PNA.

In another embodiment of the present disclosure, the second strand may form a complementary bond with the first strand in a region other than the binding site with PNA.

In still another embodiment of the present disclosure, the second strand of the RNA molecule may have a 3'-overhang composed of 1 to 5 nucleotides.

In still another embodiment of the present disclosure, the RNA molecule may have chemical modification at least one of the sugar structure of a ribonucleotide, the base structure of a ribonucleotide and a binding site between the ribonucleotide.

In still another embodiment of the present disclosure, the chemical modification may be that the OH group at the 2' position of a ribonucleotide is substituted with H, OR, R, halogen, SH, SR, NH₂, NHR, NR₂, or CN, wherein R is C₁-C₆ alkyl, alkenyl or alkynyl.

In still another embodiment of the present disclosure, the chemical modification may substitute the phosphodiester bond of the backbone with boranophosphate or phosphorothioate.

In still another embodiment of the present disclosure, the target nucleic acid may be any one selected from the group consisting of messenger RNA (mRNA), microRNA, piwi-interacting RNA (piRNA), non-coding RNA (ncRNA), a coding DNA sequence, and a non-coding DNA sequence.

In still another embodiment of the present disclosure, the nucleic acid molecule may have improved *in vivo* stability.

In addition, the present disclosure provides a complex for inducing RNAi, including the nucleic acid molecule, and an intracellular carrier.

In an embodiment of the present disclosure, the intracellular carrier may be selected from the group consisting of cationic polymers, lipids, cell penetrating peptides, and cell targeting ligands.

In another embodiment of the present disclosure, the N-terminus of the cell-penetrating peptide may be linked to the C-terminus of PNA constituting the nucleic acid molecule.

In addition, the present disclosure provides a composition for gene silencing, including the complex.

In addition, the present disclosure provides a method of gene silencing, including treating the complex to cells.

In addition, the present disclosure provides a use of the complex for gene silencing.

### ADVANTAGEOUS EFFECTS OF DISCLOSURE

A nucleic acid molecule for inducing RNAi according to the present disclosure is capable of significantly improving stability of a double-stranded RNA molecule *in vivo* while maintaining inhibition efficiency for a target gene, and therefore, as a expandable platform for targeting various genes, the RNA nucleic acid molecule is expected to replace siRNA molecules in the art, and be usefully utilized in the field of diagnosis or treatment of various diseases in research and clinical fields.

### BRIEF DESCRIPTION OF DRAWINGS

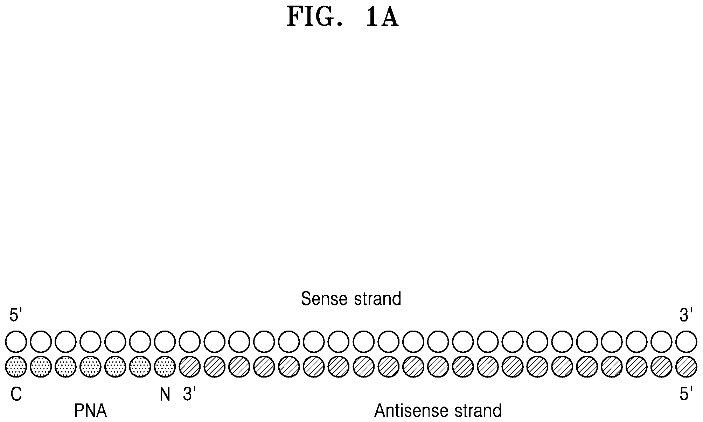

FIG. 1A shows a diagram illustrating a structure of a PNA-siRNA nucleic acid molecule synthesized in an example of the present disclosure, wherein in the structure, the N-terminus of PNA is connected to the 3' end of an antisense strand of siRNA and forms a complementary bond with the 5' end of a sense strand.

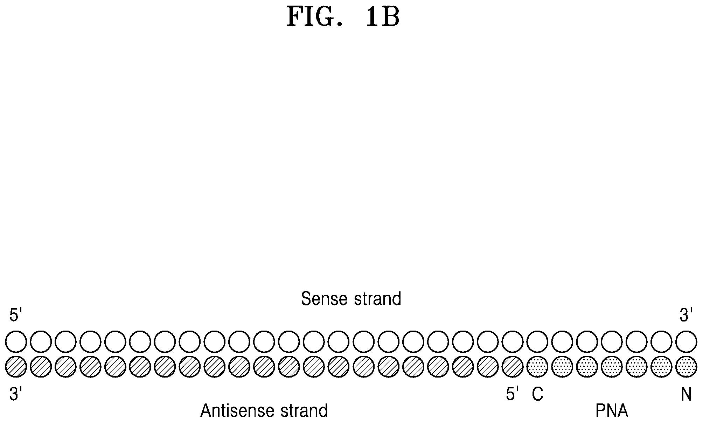

FIG. 1B shows a diagram illustrating a structure of a PNA-siRNA nucleic acid molecule synthesized in an example of the present disclosure, wherein in the structure, the C-terminus of PNA is connected to the 5' end of an antisense strand of the siRNA, and forms a complementary bond with the 3' end of a sense strand.

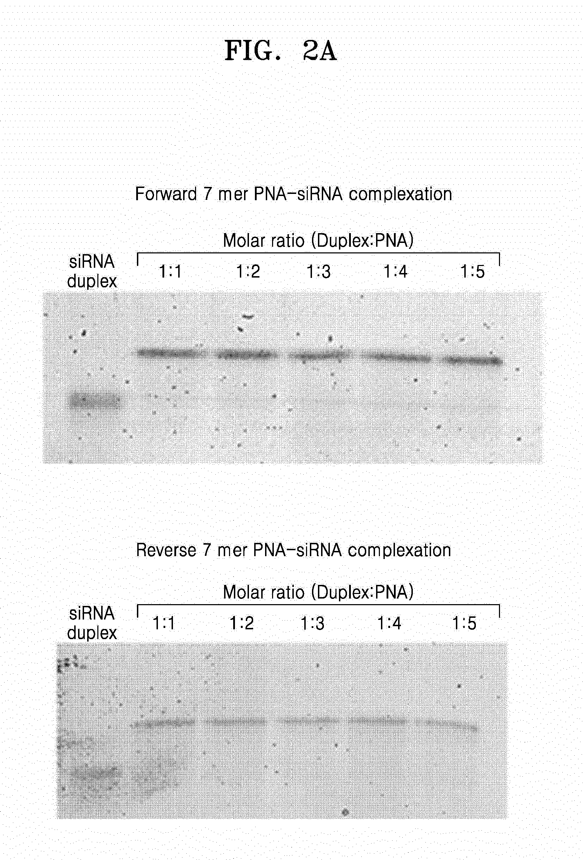

FIG. 2A shows results of verifying complexation when siRNA duplexes and 7 mer PNAs are mixed at various molar concentration ratios (1:1 to 1:5), and analyzing complexation of the structures of FIG. 1A (Forward) and FIG. 1B (Reverse).

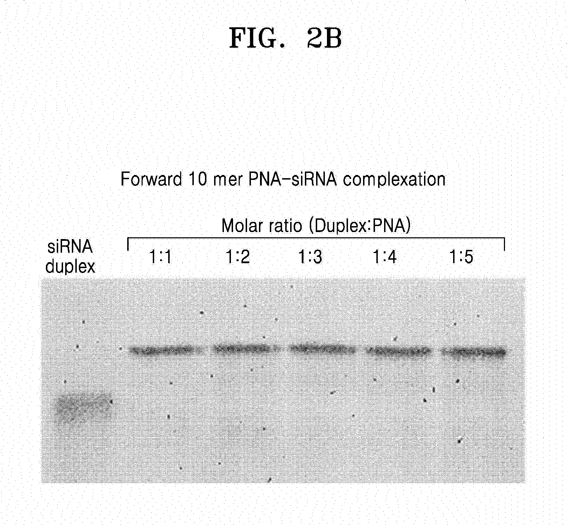

FIG. 2B shows results of verifying complexation when siRNA duplexes and 10 mer PNAs are mixed at various molar concentration ratios (1:1 to 1:5).

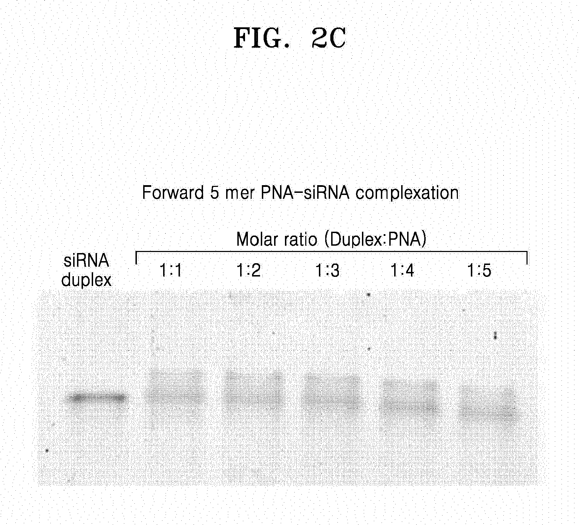

FIG. 2C shows results of verifying complexation when siRNA duplexes and 10 mer PNAs are mixed at various molar concentration ratios (1:1 to 1:5).

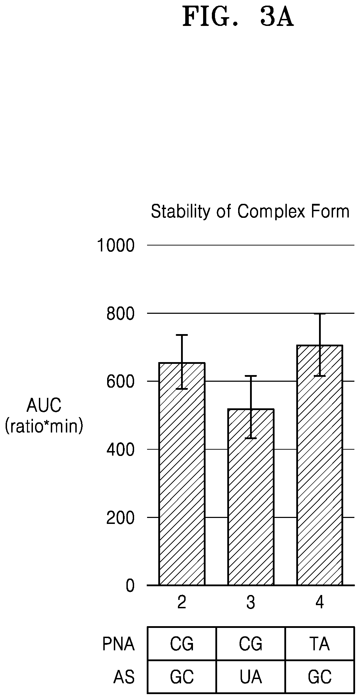

FIG. 3A shows results of analyzing serum stability through performing native polyacrylamide gel electrophoresis (PAGE) for PNA-siRNA nucleic acid molecules according to the present disclosure, and shows results of quantifying intensities of the bands after performing native PAGE for the PNA-siRNA complex form.

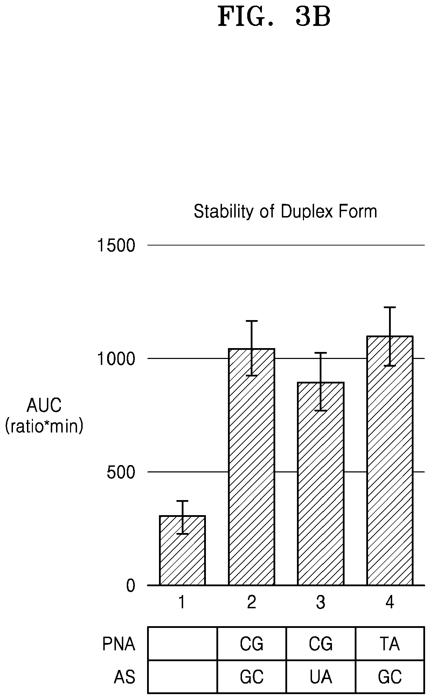

FIG. 3B shows results of analyzing serum stability through performing native PAGE for PNA-siRNA nucleic acid molecules according to the present disclosure, and shows results of quantifying intensities of the bands after performing native PAGE for the siRNA duplex form from which PNA is separated.

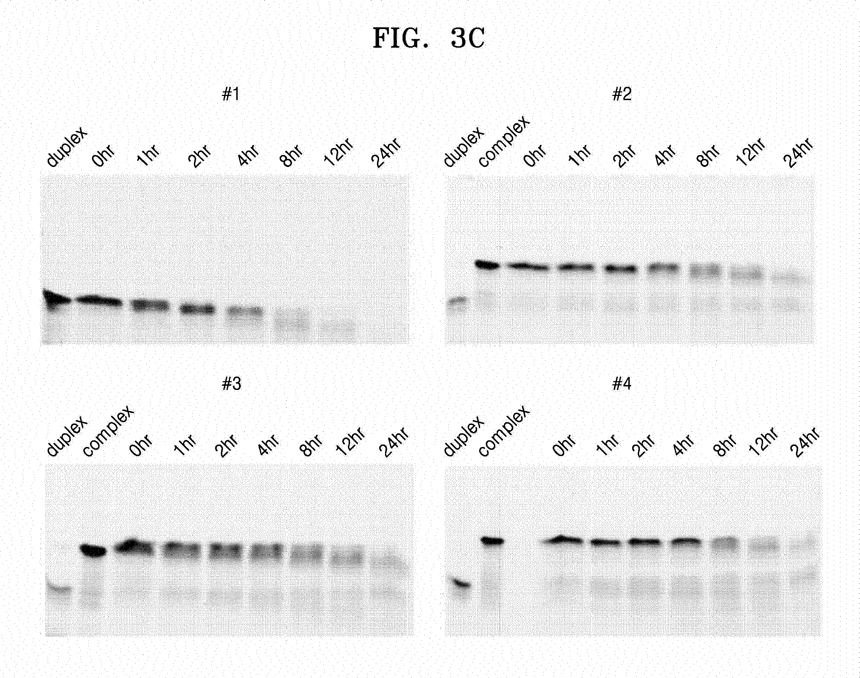

FIG. 3C shows gel images after performing native PAGE in the analyses from which FIGS. 3A and 3B are obtained.

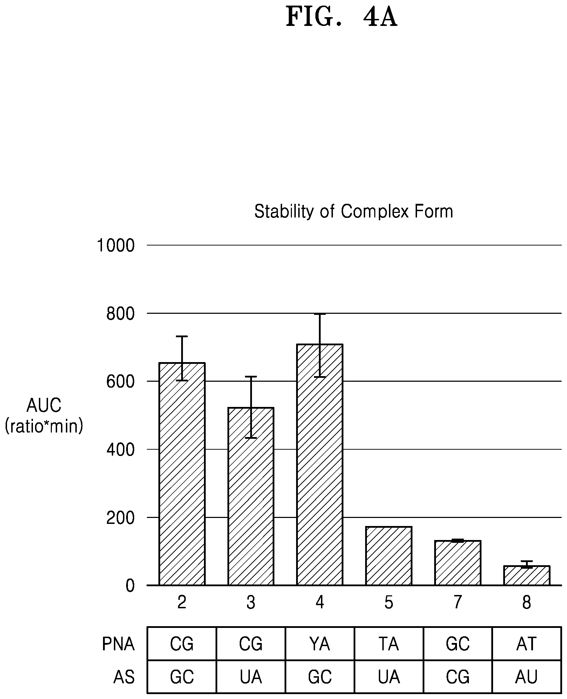

FIG. 4A shows results of analyzing serum stability according to a position of PNA in PNA-siRNA nucleic acid molecules (the structures of FIGS. 1A and 1B), and a difference in the nucleotide sequence of a binding region at which the PNA and an antisense strand are linked; and shows results of quantifying intensities of the bands after performing native PAGE for the PNA-siRNA complex form.

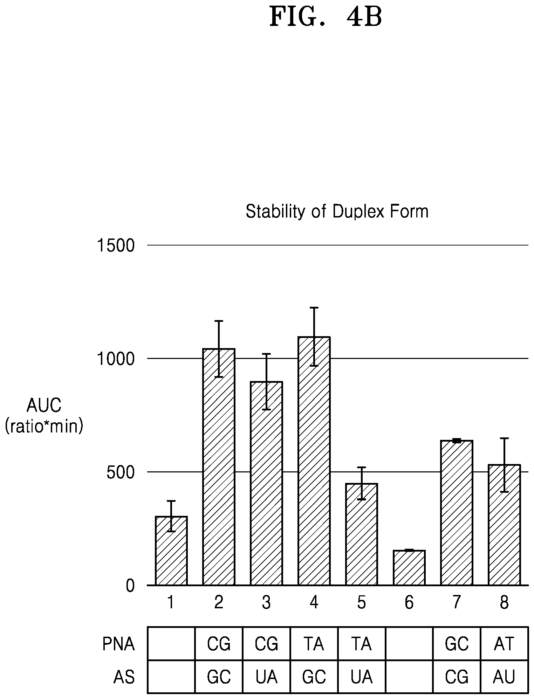

FIG. 4B shows results of analyzing serum stability according to a position of PNA in PNA-siRNA nucleic acid molecules (the structures of FIGS. 1A and 1B) and a difference in the nucleotide sequence of a binding region at which the PNA and an antisense strand are linked; and also shows results of quantifying intensities of the bands after performing native PAGE for the siRNA duplex form from which PNA is separated.

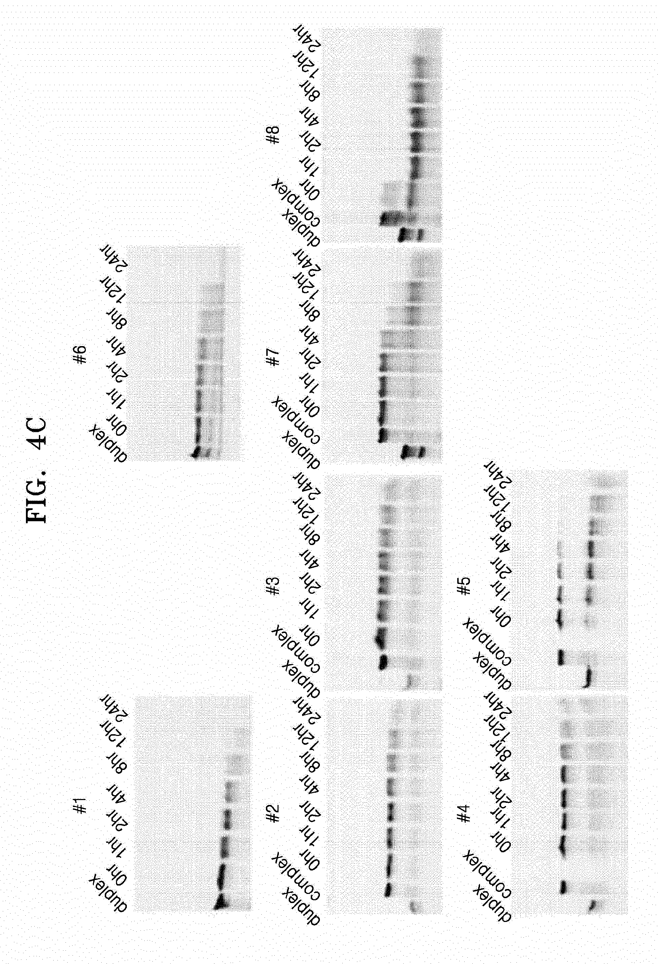

FIG. 4C shows gel images after performing native PAGE in the analyses from which FIGS. 4A and 4B are obtained.

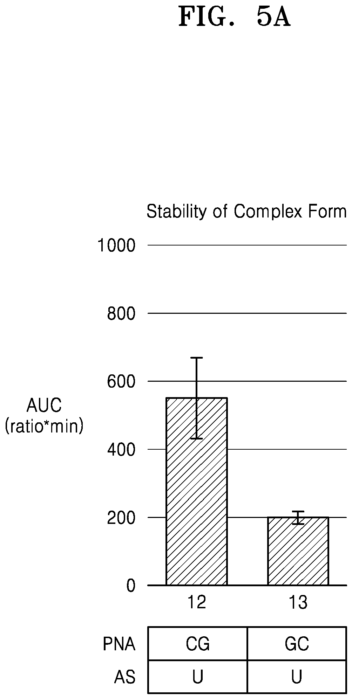

FIG. 5A shows results of analyzing serum stability according to a difference in PNA positions by using nucleic acid molecules respectively having the structure of FIG. 1A (#12) and the structure of FIG. 1B (#13), in which the sequences of the binding regions where the PNA and an antisense strand are linked are the same but, positions of the PNA are different; and also shows results of quantifying intensities of the bands after performing native PAGE for the PNA-siRNA complex form.

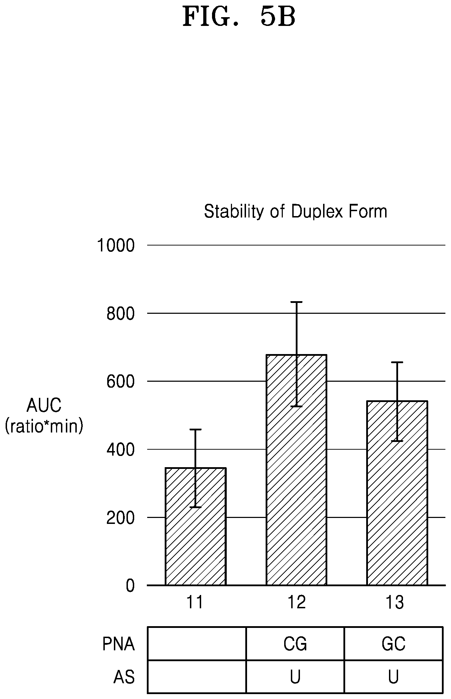

FIG. 5B shows results of analyzing serum stability according to a difference in PNA positions by using nucleic acid molecules respectively having the structure of FIG. 1A (#12) and the structure of FIG. 1B (#13), in which the sequences of the binding regions where the PNA and an antisense strand are linked are the same but, positions of the PNA are different; and also shows results of quantifying intensities of the bands after performing native PAGE for the siRNA duplex form from which PNA is separated.

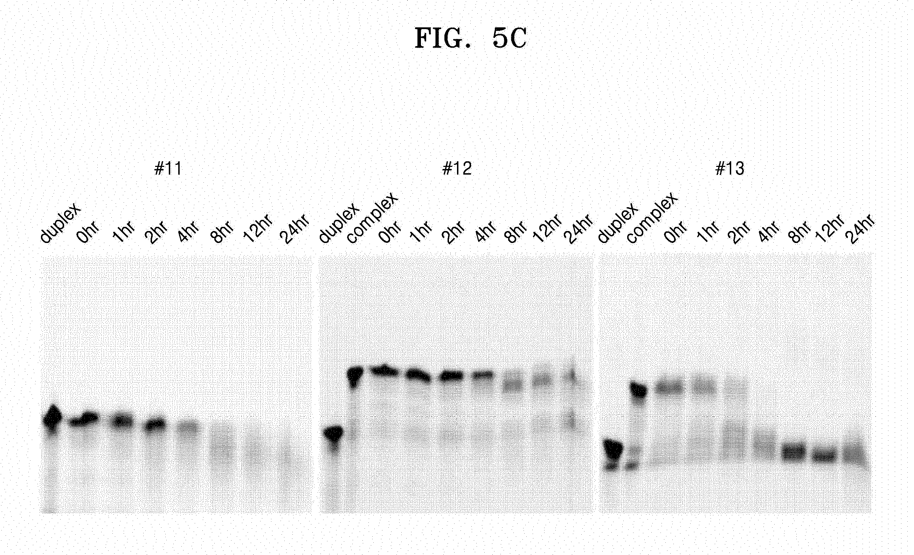

FIG. 5C shows gel images after performing native PAGE in the analyses from which FIGS. 5A and 5B are obtained.

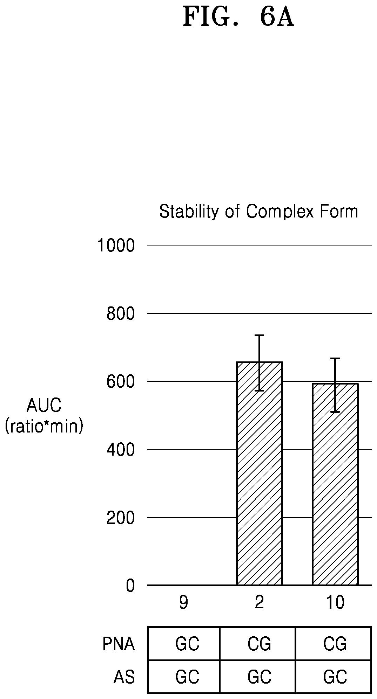

FIG. 6A shows results of analyzing serum stability according to a length (5, 7, 10 mer) of PNA in the PNA-siRNA nucleic acid molecule having the same structure as the structure corresponding to FIG. 1A; and also shows results of quantifying intensities of the bands after performing native PAGE for the PNA-siRNA complex form.

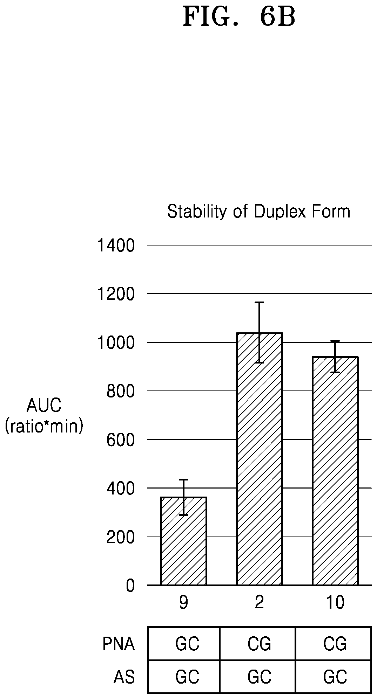

FIG. 6B shows results of analyzing serum stability according to a length (5, 7, 10 mer) of PNA in the PNA-siRNA nucleic acid molecule having the same structure as the structure corresponding to FIG. 1A; and also shows results of quantifying intensities of the bands after performing native PAGE for the siRNA duplex form from which PNA is separated.

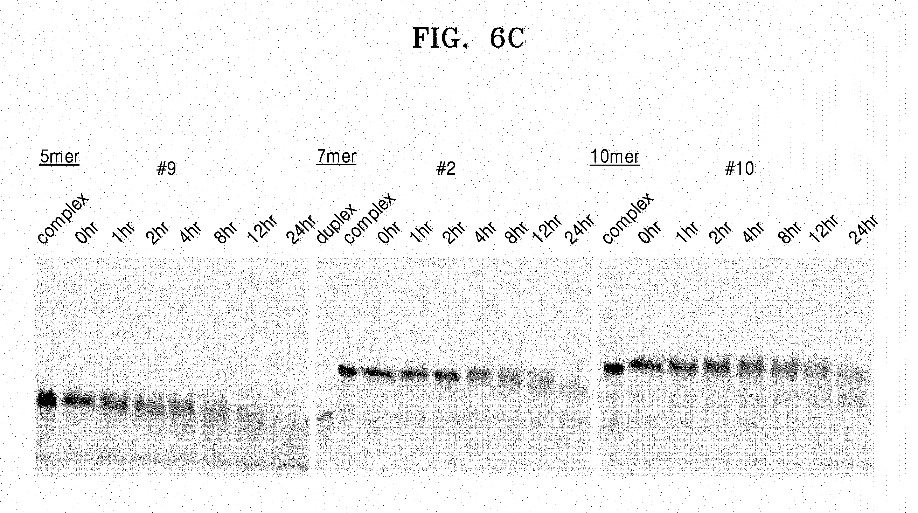

FIG. 6C shows gel images after performing native PAGE in the analyses from which FIGS. 6A and 6B are obtained.

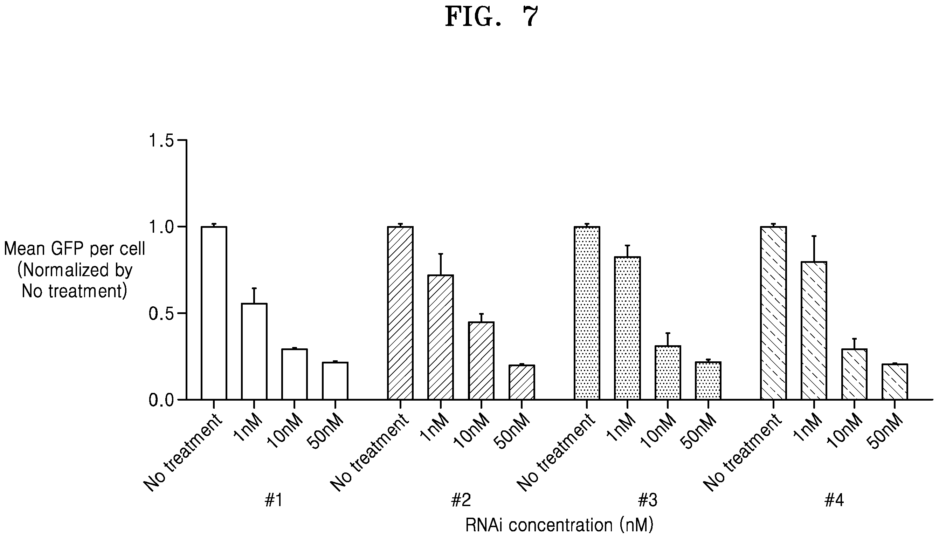

FIG. 7 shows results of analyzing *in vitro* gene silencing efficiency to analyze an effect of a PNA-siRNA nucleic acid molecule on the gene silencing efficiency of siRNA.

### MODE OF DISCLOSURE

The present disclosure relates to an RNA nucleic acid molecule that may be usefully utilized in the field of research, diagnosis or treatment of various diseases, etc, and relates to a basic platform RNA nucleic acid molecule structure that is expandable to an unlimited number of designs.

Hereinafter, the present disclosure will be described in more detail.

The present disclosure provides a nucleic acid molecule for inducing RNAi, including: a double-stranded RNA molecule consisting of a first strand, which is 15 to 49 nucleotides in length and including a region complementary to a target nucleic acid, and a second strand, which is 21 to 62 nucleotides in length and forming a complementary bond with the first strand; and a peptide nucleic acid (PNA), which is 6 to 13 nucleotides in length and bound to the first strand, wherein
the N-terminus of the PNA is joined to the 3' end of the first strand, and
the binding region between the PNA and the first strand consists of guanine (G) or cytosine (C) bases.

The term "RNA interference (RNAi)", used herein, refers to a mechanism that regulates expression of a target gene by inducing mRNA degradation of the target gene in a sequence-specific manner, by a double-stranded RNA (dsRNA) composed of a strand having a sequence homologous to mRNA of a target gene, and another strand having a sequence complementary the first strand.

In the present disclosure, the double-stranded RNA has a function of targeting a target nucleic acid and inhibiting its expression, and may preferably be siRNA.

The term "small interfering RNA (siRNA)", used herein, refers to a short double-stranded RNA (dsRNA) that mediates efficient gene silencing by inducing an RNAi phenomenon by sequence-specifically cleaving mRNA of a target gene. The double-strand is composed of a sense RNA strand, and an antisense RNA strand having a sequence complementary to the sense RNA strand. Since siRNA suppresses expression of a target gene, it may be used as an efficient gene knock-down method or as a means of gene therapy.

The first strand refers to an antisense strand, and is a polynucleotide at least 50 %, preferably 70 %, more preferably at least 85 %, and most preferably 100 % complementary to the target nucleic acid of interest. For example, the first strand may be complementary, in whole or in part, to messenger RNA (mRNA), an RNA sequence which is not mRNA (for example, microRNA, piwiRNA, tRNA, rRNA, and hnRNA), or a coding or non-coding DNA sequence.

In the present disclosure, the first strand may consist of 15 to 49 nucleotides, preferably 15 to 39 nucleotides, more preferably 15 to 30 nucleotides that include a region complementary to a target nucleic acid, however, a length of the first strand of the RNA molecule specific to a target nucleic acid may be adjusted without limitation within the above range, depending on the target nucleic acid.

The second strand refers to a sense strand, a polynucleotide having the same nucleic acid sequence as a target nucleic acid, and a polynucleotide identical, in whole or in part, to messenger RNA (mRNA), an RNA sequence which is not mRNA (for example, microRNA, piwiRNA, tRNA, rRNA, and hnRNA), or a coding or non-coding DNA sequence.

In the present disclosure, the second strand may consist of 21 to 62 nucleotides, preferably 21 to 52 nucleotides, more preferably 21 to 43 nucleotides that forms a complementary bond with the first strand, however, a length of the second strand of the RNA molecule specific to a target nucleic acid may be adjusted without limitation within the above range, depending on the target nucleic acid.

In the present disclosure, the binding region may include at least one region selected from the group consisting of a region of 1 to 2 nucleotides from the 3' end of the first strand, and a region of 1 to 2 nucleotides from the N-terminus of the PNA, or more preferably, may include a region of 2 nucleotides.

In the present disclosure, the second strand may form a complementary bond with the first strand in a region other than the portion that forms a complementary bond with PNA. Specifically, all nucleotides constituting the nucleic acid molecule for inducing RNAi of the present disclosure may form a complementary binding pair, so that both ends of the nucleic acid molecule may form blunt ends.

In the present disclosure, the second strand of the RNA molecule may have a 3'-overhang composed of 1 to 5 nucleotides, preferably a 3'-hydroxy group.

In the present disclosure, the 5' end of at least one of the two strands of the RNA molecule may have a phosphate, a diphosphate, a triphosphate, or a hydroxyl group.

In the present disclosure, the RNA molecule may have a natural (unmodified) ribonucleotide unit structure, or may be chemically modified, for example, synthesized to have one or more chemical modifications. Effects that may be obtained through the chemical modification include enhancement of resistance to nucleases, increase of uptake into cells, improvement of cell targeting (target specificity), increase of stability, decrease of interferon activity, reduction of off-target effects such as immune responses and sense effects, and increase in RNA-induced silencing complex (RISC) loading (increase in RNAi activity).

In the present disclosure, a method of chemical modification of the RNA molecule is not particularly limited, and for example, the sugar structure or base structure of one or more ribonucleotides, or the binding site between the ribonucleic acids may be chemically modified, and those of ordinary skill in the art may synthesize and modify the RNA molecule in a desired manner using methods known in the art. In addition, the modifications may also include combined forms.

In the present disclosure, the chemical modification may include a nucleobase-modified ribonucleotide, that is, uridine or cytidine modified at position 5, for example, 5-(2-amino)propyl uridine, 5-bromo uridine; adenosine and guanosine modified at position 8, such as 8-bromo guanosine; deaza nucleotides such as 7-deaza-adenosine; O- and N-alkylated nucleotides, for example, ribonucleotides including unnatural nucleobases instead of natural nucleobases, such as N6-methyl adenosine.

In addition, in sugar-modified ribonucleotides, the 2'OH group may be substituted by a substituent selected from the group consisting of H, OR, R, halogen (F, Cl, Br, or I), SH, SR, NH₂, NHR, NR₂, and CN (R is C₁-C₆ alkyl, alkenyl, or alkynyl).

In addition, in a ribonucleotide, the phosphodiester bond of the backbone may be substituted with boranophosphate or phosphorothioate.

The term "target nucleic acid", used herein, refers to a nucleic acid that forms a complementary bond with an antisense strand of siRNA in the nucleic acid molecule of the present disclosure, and preferably refers to a target nucleic acid whose expression is to be inhibited by siRNA, or more preferably refers to any one selected from the group consisting of messenger RNA (mRNA), microRNA, piwi-interacting RNA (piRNA), non-coding RNA (ncRNA), coding DNA sequence, and non-coding DNA sequence, but is limited thereto.

In the present disclosure, the nucleic acid molecule may be synthesized in a general way, but is not limited thereto. That is, in the present disclosure, the nucleic acid molecule may be chemically or enzymatically synthesized. The double-stranded RNA molecule of the present disclosure may be derived from a naturally occurring gene by standard recombination techniques, but in this case, the double-stranded RNA may be characterized by being substantially complementary to at least a part of the mRNA of the target gene whose expression is to be changed, at the nucleotide sequence level.

The present disclosure confirmed enhanced *in vivo* stability effect of the nucleic acid molecule for inducing RNAi according to the present disclosure through specific examples.

In an embodiment of the present disclosure, RNA nucleic acid molecules of the structure according to the present disclosure and control RNA nucleic acid molecules for comparison of stability effects thereof were designed and synthesized (see Example 2).

In another example of the present disclosure, it was confirmed that 7 mer and 10 mer PNA constituting the RNA nucleic acid molecule designed above and double-stranded RNA easily form complexes (see Example 3).

In another example of the present disclosure, enhanced stability effect of the RNA nucleic acid molecules according to the present disclosure was confirmed, and by comparing stability with control RNA nucleic acid molecules, it was confirmed that the structure of the nucleic acid molecules according to the present disclosure is a specific structure capable of enhancing stability. In addition, enhanced stability effect was confirmed only when a length of the PNA was 7 mer and 10 mer (see Examples 4 and 5).

In another example of the present disclosure, as a result of analyzing gene silencing efficiency of the nucleic acid molecules with the structure according to the present disclosure, it was confirmed that the gene silencing efficiency did not change even with a structural modification in which nucleotides are added (see Example 6).

The results of the examples described above demonstrate that the RNA nucleic acid molecule according to the present disclosure may significantly improve *in vivo* stability while maintaining the original gene silencing efficiency.

Accordingly, as another aspect of the present disclosure, the present disclosure provides a complex for inducing RNAi, including the nucleic acid molecule, and an intracellular carrier.

The intracellular carrier may be selected from the group consisting of cationic polymers, lipids, cell penetrating peptides, and cell targeting ligands. *Cationic cell carriers, such as the cationic polymers or cationic lipids, may be used to deliver the nucleic acid molecules of the present disclosure into a cell in any state, in vitro and in vivo.* The cationic cell carriers strongly interact with the nucleic acid molecule of the present disclosure to form a complex, so that the RNAi-inducing nucleic acid molecule may be effectively introduced into the cell. Preferably, a cationic polymer such as polyethyleneimine (PEI) or liposome may be used, but is not limited thereto. In addition, lipids such as cholesterol may be directly linked to a nucleic acid molecule or indirectly linked to a nucleic acid molecule by binding to another cell carrier and then binding to the nucleic acid molecule.

The cell-permeable peptide refers to a peptide having an ability or property to penetrate the cell (membrane) and to penetrate into the cell. The cell-penetrating peptide is not particularly limited as long as it has cell-penetrating ability, and a person skilled in the art to which the present disclosure pertains may appropriately select a cell-penetrating peptide to achieve the desired purpose. Non-limiting examples thereof include amino acid sequences represented by, or including dNP2 (KIKKVKKKGRKKIKKKVKKKGRK, SEQ ID NO: 27), Tat (RKKRRQRRR, SEQ ID NO: 28), Pep-1 (KETWWETWWTEWSQPKKKRKV, SEQ ID NO: 29), MPG (GLAFLGFLGAAGSTMGAWSQPKKKKRKV, SEQ ID NO: 30), Penetratin (RQIKIWFQNRRMKWKK, SEQ ID NO: 31), KALA(WEAKLAKALAKALAKHLAKALAKALKACEA, SEQ ID NO: 32), or CADY(GLWRALWRLLRSLWRLLWRA, SEQ ID NO: 33). In this regard, the cell-penetrating peptide may include an amino acid sequence having sequence homology of 70 % or more, preferably 80 % or more, more preferably 90 % or more, and most preferably 91 %, 92 %, 93 %, 94 %, 95 %, 96 %, 97 %, 98 %, 99 % or more, with the amino acid sequences represented by SEQ ID NOS: 27 to 33.

When the cell-penetrating peptide forms a complex for inducing RNAi with the PNA-siRNA nucleic acid molecule, the N-terminus of the peptide may be linked to the C-terminus of PNA constituting the nucleic acid molecule.

The peptide may be manufactured so that the purity of each peptide is 90 % or more by using a peptide synthesis method or a manufacturing method known to those skilled in the art, for example, the peptide may be personally synthesized, or purchased and used after requesting production from a peptide manufacturer. The peptide may be prepared in a D-form or L-form, or as a peptide in which only a part of the sequence is composed of a D-form or L-form, or in a racemate form thereof, by using a peptide synthesis method or manufacturing method known to those skilled in the art. In addition, in order to increase stability of the peptide, other modifications known in the art are possible. In the present disclosure, the peptide was preferably synthesized by using a solid phase peptide synthesis method, but as described above, the peptide synthesis method and conditions are not limited thereto.

As another aspect of the present disclosure, the present disclosure provides a composition for gene silencing, including the complex for inducing RNAi.

In the present disclosure, "gene" is to be considered in its broadest sense, and may be one encoding a structural protein or a regulatory protein, and may be associated with a pathological condition. The gene includes both endogenous genes and transgenes. A type of the gene is not specifically limited, and a person skilled in the art may appropriately select it according to the purpose.

The composition for gene silencing may additionally include a pharmaceutically acceptable carrier, and may be formulated together with the carrier. "Pharmaceutically acceptable carrier" refers to a carrier or diluent that is commonly used in formulation, which does not stimulate the organism and does not inhibit biological activity and properties of the administered material. For example, "a pharmaceutically acceptable carrier" includes, but is not limited to, saline, sterile water, Ringer's solution, buffered saline, cyclodextrin, dextrose solution, maltodextrin solution, glycerol, ethanol, liposomes, etc., and may further include other additives in the art such as antioxidants and buffers. In addition, diluents, dispersants, surfactants, binders, lubricants, etc. may be additionally added to form an injectable formulation such as an aqueous solution, suspension, emulsion, etc., or to form pills, capsules, granules, or tablets. Regarding suitable pharmaceutically acceptable carriers and formulations, formulations may be preferably made according to each component by using the methods disclosed in Remington's literature. The pharmaceutical composition of the present disclosure is not particularly limited in formulation, but may be formulated as an injection, an inhalant, or an external preparation for skin.

The composition for gene silencing of the present disclosure is applicable as any formulation including the composition as an active ingredient, and may be administered orally or parenterally (for example, by intravenous, subcutaneous, intramuscular, intraperitoneal, intradermal, intrathecal, intracerebroventricular, mucosal, or topical administration, or by inhalation) according to a desired method. Dosage may vary depending on the condition and weight of the patient, the severity of the disease, the drug form, the route and time of administration, but may be appropriately selected by those skilled in the art.

In addition, the present disclosure provides a kit for gene silencing, including the composition.

In addition, the present disclosure provides a method of gene silencing, including treating the complex for RNAi induction. The process of treating the complex preferably includes all procedures of introducing the complex into cells.

Hereinafter, preferred examples are presented to help understanding of the present disclosure. However, the following examples are only provided for easier understanding of the present disclosure, and the contents of the present disclosure are not limited by the following examples.

### [Examples]

### Example 1. Experimental method

### 1-1. PNA synthesis

In this example, PNA was synthesized by using the solid state peptide synthesis (SPPS) method, an organic synthesis method in which the C-terminus of an amino acid whose N-terminus is protected by Fmoc is combined to the N-terminus of a resin, one by one. In this regard, four types of PNA monomers, namely, Fmoc-A(Bhoc)-aeg-OH, Fmoc-G(Bhoc)-aeg-OH, Fmoc-C(Bhoc)-aeg-OH, and Fmoc-T-aeg-OH were used. All of the PNA candidates according to this example were synthesized in the same manner as above, and all reactions were carried out in a hot plate stirrer. In all reactions, N,N-dimethylformamide (DMF) was used as a solvent, and PNA coupling was performed by mixing PNA: hexafluorophosphate benzotriazole tetramethyl uronium (HBTU): N,N-diisopropylethylamine (DIEA) in a molar ratio of 1: 1: 2, followed by reacting for 1 hour. In order to synthesize the next PNA, Fmoc of the previous PNA must be removed. For this, a deprotecting process was performed at room temperature for 30 minutes by using 80 % DMF and 20 % piperidine solution. Between all coupling and deprotecting processes, the sample was washed alternately with DMF and dichloromethane (DCM) three times each. Then, in order to separate the synthesized PNA from the solid resin, after the synthesis was completed, the resin was stirred and reacted for 2 hours in a cleavage solution composed of trifluoroacetic acid (TFA): triisopropylsilane (TIS): distilled water (95: 2.5: 2.5), and then the resin was filtered through an asbestos filter. Subsequently, the solution in the filtered solution was evaporated under nitrogen gas, and when a precipitate was formed, the solution was precipitated with diethyl ether stored cold, and the precipitated PNA was dried, and then dissolved in distilled water and lyophilized. After being lyophilized, the PNA was dissolved in distilled water or acetonitrile (ACN), and separated and purified by using reverse-phase high-performance liquid chromatography (HPLC). In this regard, solvent A (distilled water 99.9 % and TFA 0.1 %) and solvent B (distilled water 9.9 %, acetonitrile 90 % and TFA 0.1 %) were used as mobile phase solvents. The HPLC mobile phase started with Solvent A 90 % and Solvent B 10 %, and the separation proceeded while the gradient of solvent B was increased at 1 %/min. The separated peptide solution was rapidly cooled in liquid nitrogen, lyophilized to remove the solvent, and then dissolved in DEPC (diethyl pyrocarbonate water) for use in the experiment.

### 1-2. Preparation of PNA-siRNA nucleic acid molecule

In order to prepare a nucleic acid molecule formed as a complex of PNA and siRNA synthesized according to the method of Example 1-1, a method of heating to a temperature higher than the melting temperature (Tm), and then cooling slowly for annealing, was used. More specifically, sense strands of siRNA: antisense strands of siRNA: PNA were mixed in a molar ratio of 1: 1: 2, annealing buffer and DEPC water were added, and then the sample was boiled for 2 minutes on a heating block heated to 100 °C. Thereafter, the temperature of the heating block was set to room temperature, and the sample was cooled slowly. The annealing buffer was used after being prepared by mixing 1 M of Tris (pH 8.0) buffer, 5 M of NaCl, and DEPC water in a volume ratio of 5: 2: 93, and making a five-fold dilution.

Complexation of PNA and siRNA was confirmed by performing native polyacrylamide gel electrophoresis (PAGE) on an 18 % native PAGE gel. Then, the result was confirmed after staining the gel for 40 minutes by using GelRed^{®}.

### 1-3. Stability analysis

For the PNA-siRNA nucleic acid molecule prepared by the method of Example 1-2, serum stability was analyzed. For this, 100 % mouse serum was used, and the PNA-siRNA nucleic acid molecule was mixed with the serum at a ratio of 1: 4 and then cultured at 37 °C. After culturing, the reaction was stopped by treating with 0.5 M of ethylenediaminetetraacetic acid (EDTA) (pH 8.0) every 0, 1, 2, 4, 8, 12, and 24 hours, and then the results were confirmed by performing native PAGE on 15 % native gel. The results were confirmed after the gel was stained for 40 minutes by using GelRed^{®}, and the same number of moles of the nucleic acid molecules not exposed to mouse serum was used as a control. Then, the intensity of the band shown on the gel image were quantified and compared with the intensity of the control band, and expressed as ratios.

### 1-4. Analysis of in vitro gene silencing efficiency

In order to examine silencing efficiency of the PNA-siRNA nucleic acid molecule prepared according to the method of Example 1-2 for GFP, a target gene, an experiment was performed according to the following method. More specifically, human 293A cells were seeded in a 384-well plate and cultured overnight at 37 °C under CO₂ conditions using DMEM medium supplemented with 10 % fetal bovine serum (FBS) and 1 % penicillin/streptomycin. Next, the cells were transfected with each of the PNA-siRNA nucleic acid molecules by using RNAiMax (Thermo fisher scientific) so as to be 1 nM, 10 nM, or 50 nM with respect to 40 µL of transfection optimized medium (TOM, TR004-01).

In addition, 48 hours after the transfection, Hoechst 33342 was diluted in DMEM to 1:5000, and then 5 µL of the diluted Hoechst 33342 was additionally added to each well and incubated for 30 minutes. Next, DAPI and GFP wavelengths were imaged at 4 points per each well by using Cytation 5 (BioTek), and then each cell was individualized based on the DAPI mask. Then, GFP values of 20 µm around the nucleus were obtained for each cell, and based on this, mean-GFP values for each well were obtained. The obtained mean-GFP values were exported to excel and expressed as a graph for comparing silencing efficiencies by using the GraphPad Prism 8 program.

### Example 2. Design and synthesis of RNA nucleic acid molecules

2-1. Design and synthesis of RNA nucleic acid molecules of the present disclosure

The present inventors designed various nucleic acid molecules satisfying the structure of the RNA molecule according to the present disclosure, and in this regard, the sequence of each RNA molecule was designed to have a melting temperature (Tm) similar to each other. Specific information on each of the RNA molecules is summarized in Table 1 below.

**[Table 1]**

| **Sam ple (#)** | **PNA (C - N) (Tm, °C)** | **Name and Sequence** |
|---|---|---|
| 1 | - | (+GC)siGFP |
| | | SS : 5'-**CG**CCCCGACCACAUGAAGCAGCA-3' (SEQ ID NO: 1) |
| | | AS : 3'-**GC**GGGGCUGGUGUACUUCGUCGU-5' (SEQ ID NO: 2) |
| 2 | ATTGC**CG** (45.2) | **PNA**-(+**GC**)siGFP |
| | | SS : 5'-UAACG**GCCG**CCCCGACCACAUGAAGCAGCA-3' (SEQ ID NO: SEQ ID NO: 3) |
| | | AS : 3'-ATTGCCGGCGGGGCUGGUGUACUUCGUCGU- 5' (SEQ ID NO: 4) |
| 3 | ATTG**CG**(45 .2) | **PNA**-(+**UA**)siGFP |
| | | SS : 5'-UAACG**GCAU**CCCCGACCACAUGAAGCAGCA-3' (SEQ ID NO: 5) |
| | | AS : 3'-ATTGCCGUAGGGGCUGGUGUACUUCGUCGU- 5' (SEQ ID NO: 6) |
| 4 | GCCGT**TA**(43 .7) | **PNA**-(+**GC**)siGFP |
| | | SS : 5'-CGGCAAU**CG**CCCCGACCACAUGAAGCAGCA-3' (SEQ ID NO: 7) |
| | | AS : 3'-GCCGTTAGCGGGGCUGGUGUACUUCGUCGU- 5' (SEQ ID NO: 8) |

As may be seen in Table 1 above, in order to synthesize the RNA molecule according to the present disclosure, various nucleic acid molecules were synthesized with the GFP-specific siRNA duplex as an example.

That is, in each RNA molecule, 4 nucleotides at the site where PNA and an antisense strand are linked, that is, 2 nucleotides from the PNA end at the linking site and 2 nucleotides from the antisense strand end, were designed to be all composed of G and C (#2), or designed so that G and C were located at only one end (#3 and #4).

In addition, in order to further diversify the structure of the RNA molecule of Table 1, RNA molecules were additionally designed as shown in Table 2 below, wherein the RNA molecules have all 4 nucleotides composed of G and C at the site where PNA and an antisense strand are linked, while the PNA is 5 mer (#9) and 10 mer (#10) in length.

**[Table 2]**

| **Sam ple (#)** | **PNA (C** - **N) (Tm, °C)** | **Name and Sequence** |
|---|---|---|
| 9 | **ATGGC** (20.2) | **PNA**-(+**GC**)siGFP |
| | | SS : 5'-UAG**GCCG**CCCCGACCACAUGAAGCAGCA-3' (SEQ ID NO: 17) |
| | | AS : 3'-***ATGGC*GC**GGGGCUGGUGUACUUCGUCGU-5' (SEQ ID NO: 18) |
| 10 | **ATTATTGCC G** (53.5) | **PNA**-(+**GC**)siGFP |
| | | SS : 5'- UAAUAACGGCCGCCCCGACCACAUGAAGCAGCA-3' (SEQ ID NO: 19) |
| | | AS : 3'- ATTATTGCCGGCGGGGCUGGUGUACUUCGUCGU-5' |
| | | (SEQ ID NO: 20) |

The RNA molecules disclosed in Tables 1 and 2 have a structure in that the N-terminus of the PNA is linked to the 3' end of the antisense strand of siRNA, and the PNA sequence is complementarily bound to the 5' end region of the sense strand of the siRNA, as pictorially shown in FIG. 1A.

In the nucleic acid molecule sequence, the PNA sequence linked to the antisense strand is shown in italics, and 4 nucleotides at the site where the PNA and the antisense strand are linked are underlined.

### 2-2. Design and synthesis of control RNA nucleic acid molecules

The present inventors designed nucleic acid molecules as controls for comparing effects with the RNA nucleic acid molecules according to the present disclosure synthesized in Example 2-1, and detailed information is shown in Table 3 below.

**[Table 3]**

| **Sam ple (#)** | **PNA (C** - **N) (Tm, °C)** | **Name and Sequence** |
|---|---|---|
| 5 | **GCCGTTA** (43.7) | **PNA**-(+**UA**)siGFP |
| | | SS : 5'-CGGCAAU**AU**CCCCGACCACAUGAAGCAGCA-3' (SEQ ID NO: 9) |
| | | AS : 3'-***GCCGT**TA*UA**GGGGCUGGUGUACUUCGUCGU-5' (SEQ ID NO: 10) |
| 6 | - | siGFP(+CG) |
| | | SS : 5'-CCCCGACCACAUGAAGCAGCA**GC**-3' (SEQ ID NO: 11) |
| | | AS : 3'-GGGGCUGGUGUACUUCGUCGU**CG**-5' (SEQ ID NO: 12) |
| 7 | **GC**CGTTA (43.7) | siGFP**(+CG)-PNA** |
| | | SS : 5'-CCCCGACCACAUGAAGCAGCA**GCCG**GCAAU-3' |
| | | (SEQ ID NO: 13) |
| | | AS : 3'-GGGGCUGGUGUACUUCGUCGU**CG*****GCCGTTA**-*5' (SEQ ID NO: 14) |
| 8 | **AT**CGTTA (33.1) | siGFP**(+AU)-PNA** |
| | | SS : 5'-CCCCGACCACAUGAAGCAGCA**UAUA**GCAAU-3' (SEQ ID NO: 15) |
| | | AS : 3'-GGGGCUGGUGUACUUCGUCGU**AU*ATCGTTA***-5' (SEQ ID NO: 16) |

In the control RNA molecules, #5 has the structure of FIG. 1A, but the 4 nucleotides at the site where PNA and an antisense strand are linked were designed to be composed of only T(U) and A.

In addition, as illustrated in FIG. 1B, RNA molecules having a structure, in which the C-terminus of PNA is linked to the 5' end of an antisense strand of siRNA, and the PNA sequence is complementarily bound to the 3' end of a sense strand of the siRNA, contrary to the structure of FIG. 1A , were designed (#7 and #8). More specifically, 4 nucleotides at the site where PNA and an antisense strand are linked, that is, 2 nucleotides from the PNA end at the linking site and 2 nucleotides from the antisense strand end, were designed to be all composed of G and C (#7), or to be all composed of T and A (#8).

### Example 3. Confirmation of complexation of PNA and double-stranded RNA

The present inventors tried to confirm whether the PNA and the double-stranded RNA designed in Examples 2-1 and 2-2, respectively, bind to form a complex.

To this end, for each of 5 mer, 7 mer, and 10 mer PNAs, complexation was observed under conditions in which the molar ratio of PNA to double-stranded RNA was varied from 1:1 to 1:5.

Specifically, PNA and siRNA duplex corresponding to #2 (7 mer), #9 (5 mer), and #10 (10 mer), the RNA nucleic acid molecules according to the present disclosure, and corresponding to #7, a control RNA nucleic acid molecule, were used. Experiments were carried out according to the method of Example 1-2, and native PAGE was performed by using 18 % native gel, and the results were observed.

As a result, as shown in FIGS. 2A to 2C , when PNA was 7 mer and 10 mer, it was found that PNA-siRNA nucleic acid molecules were formed at all of the molar ratios of 1:1 to 1:5. On the other hand, in the case of 5 mer PNA, as there was no difference in positions of the band and the band of the siRNA duplex, it was confirmed that the nucleic acid molecule was not formed, and it was judged to be due to dissociation of the 5 mer RNA having Tm of 20.2 °C.

### Example 4. Comparison of stability according to PNA position and nucleotide sequence of PNA-AS binding region

The present inventors first tried to analyze the serum stability of the RNA nucleic acid molecules according to the present disclosure, designed and synthesized in Example 2-1. For this purpose, serum stability was analyzed according to the method of Example 1-3 using nucleic acid molecules #2, #3 and #4 in Table 1, and the results were quantified and presented in a graph.

As a result, as shown in FIG. 3A, serum stability of all of the RNA nucleic acid molecules #2, #3, and #4 according to the present disclosure was significantly increased, and as may be seen in FIG. 3B, it was found that stability of the double-stranded RNA, in which PNA is separated from the nucleic acid molecule, was also maintained high. This result may also be confirmed through the intensities of the bands shown on a gel image, as shown in FIG. 3C.

Based on the above results, the present inventors, in order to determine whether the enhanced stability effect of the RNA nucleic acid molecule according to the present disclosure is specific to the structure, the stability was compared with that of the control RNA nucleic acid molecule of Table 3 synthesized in Example 2-2.

As a result, as shown in FIG. 4A, the nucleic acids (#2, #3, #4) having the structure of FIG. 1A according to the present disclosure were shown to have remarkably high stability, compared to nucleic acid molecules (#7, #8) having a structure in which the PNA of FIG. 1B is bound to the 5' end of an antisense strand of RNA, and stability of the double-stranded RNA from which PNA is separated as well as the form of the PNA-bound complexes also showed a similar level, as shown in FIG. 4B. These results may also be confirmed through the band intensities on the gel image of FIG. 4C.

In addition, the present inventors compared stability of the control nucleic acid molecule (#5), in which all four nucleotides were composed only of T(U) and A, to determine whether the nucleotide sequence of the two nucleotides at the end of PNA binding to an antisense strand of RNA and the two nucleotides at the end of an antisense strand binding to the PNA have a significant effect on stability, in the RNA nucleic acid molecule having the structure of FIG. 1A according to the present disclosure.

As a result, as shown in FIGS. 4A to 4C, unlike the nucleic acid molecules (#2, #3, #4) according to the present disclosure, in a case of the nucleic acid molecule (#5), where the 4 nucleotides were all composed of T(U) and A, stability was shown to be significantly reduced, and the result was similarly shown not only in the form of a PNA-coupled complex but also in the double-stranded RNA from which PNA was separated.

However, the present inventors found that, in the results of the PNA-bound complex of FIG. 4A, the nucleic acid molecules having the structure of FIG. 1A (#2, #3, #4, and #5) had higher stability than the nucleic acid molecules (#7, #8) of the structure of FIG. 1B, regardless of the base sequence of the site to which PNA-antisense strand binds. However, in FIG. 4B for double-stranded RNA, for nucleic acid molecules in which the PNA-antisense strand binding site is composed of T(U) and A, the nucleic acid molecule (#5) of the structure of FIG. 1A was found to have a little lower stability than the nucleic acid molecule (#8) having the structure of FIG. 1B. Accordingly, the present inventors further synthesized RNA nucleic acid molecules shown in Table 4 below in order to more accurately compare stability according to a location of PNA.

**[Table 4]**

| **Sam ple (#)** | **PNA (C - N) (Tm, °C)** | **Name and Sequence** |
|---|---|---|
| 11 | - | (+**U)**siGFP |
| | | SS : 5'-**A**CCCCGACCACAUGAAGCAGCA-3' (SEQ ID NO: 21) |
| | | AS : 3'-**U**GGGGCUGGUGUACUUCGUCGU-5' (SEQ ID NO: 22) |
| 12 | **ATTGCCG** (45.2) | **PNA**-(+**U**)siGFP |
| | | SS : 5'-UAACG**GCA**CCCCGACCACAUGAAGCAGCA-3' (SEQ ID NO: 23) |
| | | AS : 3'-***ATTGC**CG*U**GGGGCUGGUGUACUUCGUCGU-5' (SEQ ID NO: 24) |
| 13 | **GC**CGTTA (43.7) | **(+U)**siGFP-**PNA** |
| | | SS : 5'-CCCCGACCACAUGAAGCAGCAA**CG**GCAAU-3' (SEQ ID NO: 25) |
| | | AS : 3'-GGGGCUGGUGUACUUCGUCGU**U*GCCGTTA*-**5' (SEQ ID NO: 26) |

Specifically, stability according to a PNA position was more accurately compared and analyzed by using #12 and #13 nucleic acid molecules, in which other conditions such as base sequences of the PNA-antisense strand binding site, and PNA sequences were the same, but only the PNA positions were different. As a result, as shown in FIGS. 5A to 5C, stability of the RNA nucleic acid molecule (#12) having the structure of FIG. 1A according to the present disclosure was shown to be significantly high, and a similar result was also found in the double-stranded RNA form from which PNA was separated.

From the above results, it was found that the binding site of PNA and the binding region of the PNA-antisense strand of the RNA nucleic acid molecule according to the present disclosure have an important effect on stability enhancement.

### Example 5. Comparison of stability according to length of PNA

Next, in order to analyze whether a length of PNA in the RNA nucleic acid molecule affects stability of the nucleic acid molecule, and to determine the length of the PNA that may most effectively enhance stability, serum stability was analyzed according to the method of Example 1-3, by using the nucleic acid molecules #9 (PNA 5 mer), #2 (7 mer) and #10 (10 mer) of Tables 1 and 2, and the results were quantified and are shown as a graph.

As a result of the experiment, as shown in FIGS. 6A to 6C, when a length of PNA was 5 mer, an RNA molecule bound to an siRNA duplex was not formed, whereas in the case of 7 mer and 10 mer, the stability was shown to be similar, and stability of 7 mer was shown to be slightly higher. In addition, it was confirmed that even in a double stranded RNA from from which PNA was separated, stability of 7 mer and 10 mer was similar, and stability of 7 mer was a little higher.

From the above results, it may be seen that when a length of PNA is 7 mer, stability of the structure is enhanced to the highest level.

### Example 6. Confirmation of target gene silencing efficiency

The present inventors tried to determine whether an addition of nucleotides to naive siRNA for satisfying the RNA nucleic acid structure according to the present disclosure affects the original gene silencing efficiency. For this purpose, gene silencing efficiency of the RNA nucleic acid molecules (#2, #3 and #4) of the present disclosure was analyzed according to the method of Examples 1-4 using GFP siRNA duplex (#1) in a form PNA is not bound as a control.

As a result, as shown in FIG. 7, the RNA nucleic acid molecules were found to maintain gene silencing efficiency of siRNA, despite having an altered structure compared to the control.

Through this, it was confirmed that the RNA nucleic acid molecule according to the present disclosure may significantly improve *in vivo* stability while maintaining gene silencing efficiency of double-stranded RNA.

The above description of the present disclosure is for illustrative purposes, and those skilled in the art to which the present disclosure belongs will be able to understand that the examples and embodiments can be easily modified without changing the technical idea or essential features of the disclosure. Therefore, it should be understood that the above examples are not limitative, but illustrative in all aspects.

### INDUSTRIAL APPLICABILITY

The nucleic acid molecule for RNAi induction according to the present disclosure is an expandable platform that targets various genes, and has remarkably excellent *in vivo* stability, and thus, is expected to be usefully utilized in the field of diagnosis or treatment of various diseases in research or clinical practice.

## Claims

1. A nucleic acid for inducing RNAi, the nucleic acid comprising:
a double-stranded RNA molecule consisting of a first strand, which is 15 to 49 nucleotides in length and including a region complementary to a target nucleic acid, and a second strand, which is 21 to 62 nucleotides in length and forming a complementary bond with the first strand; and
a peptide nucleic acid (PNA), which is 6 to 13 nucleotides in length and bound to the first strand, wherein
the N-terminus of the PNA is joined to the 3' end of the first strand, and
the binding region between the PNA and the first strand consists of guanine (G) or cytosine (C) bases.

2. The nucleic acid of claim 1, wherein the binding region comprises at least one region selected from the group consisting of a region of 1 to 2 nucleotides from the 3' end of the first strand, and a region of 1 to 2 nucleotides from the N-terminus of the PNA.

3. The nucleic acid of claim 1 or claim 2, wherein the second strand forms a complementary bond with the first strand in a region other than the binding site with PNA.

4. The nucleic acid of claim 1 or claim 2, wherein the second strand of the RNA molecule comprises a 3'-overhang composed of 1 to 5 nucleotides.

5. The nucleic acid of claim 1 or claim 2, wherein the RNA molecule has chemical modification at least one of the sugar structure of a ribonucleotide, the base structure of a ribonucleotide and a binding site between the ribonucleotide.

6. The nucleic acid of claim 5, wherein the chemical modification is that the OH group at the 2' position of a ribonucleotide is substituted with H, OR, R, halogen, SH, SR, NH₂, NHR, NR₂, or CN, wherein R is C₁-C₆ alkyl, alkenyl or alkynyl.

7. The nucleic acid of claim 5, wherein the chemical modification substitutes the phosphodiester bond of the backbone with boranophosphate or phosphorothioate.

8. The nucleic acid of claim 1 or claim 2, wherein the target nucleic acid is any one selected from the group consisting of messenger RNA (mRNA), microRNA, piwi-interacting RNA (piRNA), non-coding RNA (ncRNA), a coding DNA sequence, and a non-coding DNA sequence.

9. The nucleic acid of claim 1 or claim 2, wherein the nucleic acid molecule has improved *in vivo* stability.

10. A complex for inducing RNAi, the complex comprising:
the nucleic acid molecule of claim 1 or claim 2; and
an intracellular carrier.

11. The complex of claim 10, wherein the intracellular carrier is selected from the group consisting of cationic polymers, lipids, cell penetrating peptides, and cell targeting ligands.

12. The complex of claim 11, wherein the N-terminus of the cell-penetrating peptide is linked to the C-terminus of PNA constituting the nucleic acid molecule.

13. A composition for gene silencing, the composition comprising the complex of claim 10.

14. A method of gene silencing, the method comprising treating the complex of claim 10 to cells.
